# EUROPÄISCHE PATENTSCHRIFT

(11) **EP 4 192 398 B1**
(45) Veröffentlichungstag und Bekanntmachung des Hinweises auf die Patenterteilung: **14.10.2026**
(21) Anmeldenummer: 21762366.9
(22) Anmeldetag: 03.08.2021
(51) Int. Cl.: A61F 2/66, A61F 2/50, A61F 2/68

(54) **PROTHESENFUSS**
PROSTHETIC FOOT
PIED PROTHÉTIQUE

(30) Priorität: 04.08.2020 DE 102020120498
(43) Veröffentlichungstag der Anmeldung: 14.06.2023
(73) Patentinhaber: Ottobock SE & Co. KGaA, 37115 Duderstadt (DE)
(72) Erfinder: MÖNICKE, Carsten, 37115 Duderstadt (DE); MEYER, Hermann, 37073 Göttingen (DE); KARSTENS, Martin, 37073 Göttingen (DE); KRENZ, Hannes, 01309 Dresden (DE); OTT, Tobias, 37339 Wehnde (DE); KUBITZ, Sven, 37083 Göttingen (DE); SCHWARTZ, Miclas, 37083 Göttingen (DE)
(74) Vertreter: Gramm, Lins & Partner Patent- und Rechtsanwälte PartGmbB
(86) Internationale Anmeldenummer: PCT/EP2021/071650
(87) Internationale Veröffentlichungsnummer: WO 2022/029114

(56) Entgegenhaltungen:
- EP-B1- 3 137 020
- CN-A- 111 084 682
- US-A- 5 888 239
- US-B2- 7 862 622
- US-B2- 8 048 172

## Beschreibung

Die Erfindung betrifft einen Prothesenfuß mit einem Fußteil und einem Unterschenkelteil, das um eine Schwenkachse schwenkbar mit dem Fußteil verbunden und in verschiedenen Schwenkwinkeln relativ zu dem Fußteil arretierbar ist, wobei die Verbindung zwischen dem Unterschenkelteil und dem Fußteil mit einem Spiel in Schwenkrichtung behaftet ist, wenn das Fußteil an dem Unterschenkelteil arretiert ist, wobei der Prothesenfuß wenigstens ein Reibelement aufweist, das zwischen dem Fußteil und dem Unterschenkelteil an der Schwenkachse angeordnet ist und ein Reibmoment aufbringt, das einem Verschwenken des Fußteiles relativ zu dem Unterschenkelteil entgegenwirkt, wenn das Fußteil relativ zu dem Unterschenkelteil arretiert ist.

Ein derartiger Prothesenfuß ist beispielsweise aus der CN 111 084 682 A bekannt. Ähnliche Ausgestaltungen sind der EP 3 137 020 B1 zu entnehmen. In der US 5,888,239 wird ein Prothesenfuß beschrieben, bei dem die beiden miteinander verbundenen Bauteile über Formschlusselemente ineinander eingreifen. Die beiden Bauteile werden miteinander verschraubt und so aufeinander gedrückt. Eine Bewegung ist nicht möglich. Die US 8,048,172 B2 geht einen anderen Weg und beschreibt einen Aktuator, der derart gehemmt werden kann, dass eine Bewegung unmöglich ist.

Ein gattungsgemäßer Prothesenfuß ist beispielsweise aus der DE 10 2014 010 938 A1 bekannt. Derartige Prothesenfüße verfügen insbesondere über eine Absatzhöheneinstellung. Das Fußteil kann in unterschiedlichen Schwenkwinkeln relativ zu dem Unterschenkelteil arretiert werden. Damit ist beispielsweise ein Winkel zwischen einer Fußsohle, also insbesondere der Kontaktfläche des Prothesenfußes, mit der der Prothesenfuß mit dem Boden in Kontakt kommen soll, und dem Unterschenkelteil oder einem an dem Unterschenkelteil angeordneten Unterschenkel einstellbar. Dies ist insbesondere von Vorteil, wenn der Träger des Prothesenfußes unterschiedliche Schuhe mit unterschiedlichen Absatzhöhen tragen möchte. Je höher der Absatz ist, desto steiler muss das Fußteil gestellt werden.

Absatzhöhenverstellungen sind seit langem bekannt und im Stand der Technik etabliert. Vorzugsweise sind die entsprechenden Ausgestaltungen derart ausgebildet, dass eine Verstellung der Absatzhöhe möglichst einfach möglich ist, damit der Träger des Prothesenfußes ohne fremde Hilfe und bevorzugt ohne Werkzeug schnell eine entsprechende Einstellung vornehmen kann, wenn beispielsweise die Schuhe gewechselt werden sollen.

Aus dem Stand der Technik sind unterschiedliche Mechanismen bekannt, die verwendet werden, um das Fußteil an dem Unterschenkelteil in unterschiedlichen Schwenkwinkeln zu arretieren. In einer besonders einfachen Ausgestaltung verfügen sowohl das Unterschenkelteil als auch das Fußteil über mehrere Bohrungen, die in unterschiedlichen Positionen und Orientierungen der beiden Bauteil miteinander in Überdeckung gebracht werden können. In diesen Positionen und Orientierungen wird dann beispielsweise ein Stift oder Bolzen durch die Bohrungen geschoben, so dass die beiden Bauteile relativ zueinander arretiert sind. Derartige Verbindungen sind aufgrund der Fertigungstoleranzen mit einem Spiel in Schwenkrichtung ausgestattet, weil in der Regel der Durchmesser des Stiftes oder Bolzens etwas kleiner dimensioniert ist als der Durchmesser der Bohrung. Die Schwenkachse, mit der eine Absatzhöhe einstellbar ist, verläuft zumindest nahezu, bei den meisten Prothesenfüßen aus dem Stand der Technik jedoch genau von medial nach lateral, also quer zur Laufrichtung des Prothesenfußes.

Während eines Schrittes wird der Prothesenfuß zunächst mit der Ferse aufgesetzt. Dieser sogenannte "Fersenauftritt" oder "heelstrike" ist der Beginn der Standphase des Schrittes, in der der Prothesenfuß mit dem Boden in Kontakt ist. Durch die Belastung des Prothesenfußes auf der Ferse wird ein Drehmoment um die Schwenkachse erzeugt, das in Richtung Plantarflexion wirkt. Eine Plantarflexion ist das Absenken der Zehen und des Vorfußbereichs. Im Laufe der Standphase verschiebt sich der Belastungspunkt in Richtung Vorfuß des Prothesenfußes (nach frontal), bis zu dem Moment, zu dem die Zehen den Boden verlassen ("toe-off") und der gleichzeitig das Ende der Standphase definiert. In diesem Moment ist der Prothesenfuß nur noch im Vorfußbereich belastet und es entsteht ein Drehmoment, das in Richtung Dorsalflexion wirkt. Die Dorsalflexion ist die der Plantarflexion entgegengesetzte Bewegung und beschreibt das Anheben des Vorfußes und der Zehen in Richtung Unterschenkel.

Durch das vorhandene Spiel der Verbindung zwischen Fußteil und Unterschenkelteil kann das Fußteil minimal relativ zu dem Unterschenkelteil verschwenkt werden, wenn die beiden Bauteile relativ zueinander arretiert sind. Zu Beginn einer Standphase wird daher das Fußteil relativ zu dem Unterschenkelteil in Plantarflexionsrichtung verschwenkt, bis es anschlägt. Im Verlauf des Schrittes wechselt das Drehmoment das Vorzeichen und das Fußteil wird relativ zu dem Unterschenkelteil in Dorsalflexionsrichtung verschwenkt, bis es anschlägt. In einem Schritt kommt es folglich zu zwei harten Anschlägen, die ein durchaus hörbares Geräusch entwickeln. Dies wird als nachteilig empfunden, beispielsweise weil es den Prothesenträger als solchen verrät.

Im Stand der Technik sind daher verschiedene Methoden vorgeschlagen worden, diese Bewegungen aufgrund des Spiels zu unterbinden. Aus der US 8,317,875 B2 beispielsweise ist ein gattungsgemäßer Prothesenfuß bekannt, bei dem das Unterschenkelteil mit einem federbelasteten Stift druckbeaufschlagt wird, der eine so große Kraft aufbringt, dass die Belastungen, die beim Gehen auftreten, nicht ausreichen, um eine Relativbewegung zwischen Fußteil und Unterschenkelteil hervorzurufen. Aus der US 7,416,565 B1 hingegen ist eine Befestigung zwischen Fußteil und Unterschenkelteil bekannt, die mittels einer Schlingfeder ein Spiel verhindert.

Nachteilig bei diesen Lösungen ist, dass durch die jeweiligen Ausgestaltungen ein Lösen der Arretierung zwischen dem Fußteil und dem Unterschenkelteil erschwert wird. Gleiches gilt beispielsweise auch für Ausgestaltungen, bei denen in die bereits beschriebenen Bohrungen kein Stift oder Bolzen, sondern beispielsweise eine Schraube hindurchgeführt ist und mittels einer Mutter das Fußteil und das Unterschenkelteil so gegeneinander verspannt werden, dass eine spielbedingte Bewegung verhindert wird. Auch in diesem Fall ist das Lösen der Arretierung erschwert, da die Verspannung gelöst werden muss. Dies ist ohne Werkzeug und gegebenenfalls ohne fremde Hilfe nicht oder nur sehr schwer möglich.

Der Erfindung liegt daher die Aufgabe zugrunde, einen Prothesenfuß so zu verbessern, dass das Fußteil relativ zu dem Unterschenkelteil leicht gelöst und in anderer Position und/oder Orientierung wieder arretiert werden kann, und bei dem die Nachteile aus dem Stand der Technik behoben oder zumindest abgemildert sind.

Die Erfindung löst die gestellt Aufgabe durch einen Prothesenfuß gemäß dem Oberbegriff des Anspruchs 1, der sich dadurch auszeichnet, dass das wenigstens eine Reibelement um die Schwenkachse herum angeordnet ist.

Durch die erfindungsgemäße Ausgestaltung des Prothesenfußes wird ein Reibmoment aufgebracht, sobald eine durch das Spiel in der Verbindung zwischen dem Fußteil und dem Unterschenkelteil ermöglichte Verschwenkung stattfindet. Dieses Reibmoment wird mit anderen Worten also immer dann aufgebracht, wenn das Fußteil relativ zu dem Unterschenkelteil verschwenkt wird, wenn die beiden Bauteile relativ zueinander arretiert sind. Diese Bewegung ist nur durch das vorhandene Spiel möglich und in ihrem Umfang auch durch das Spiel begrenzt. Durch dieses Reibmoment wird die Bewegung nicht vollständig unterbunden. Vielmehr wird sie verlangsamt, so dass der Anschlag der beiden Bauteile aneinander, der auch bei der erfindungsgemäßen Ausgestaltung des Prothesenfußes stattfindet, abgemildert wird. Das bedeutet, die beiden Bauteile bewegen sich im Vergleich zum Stand der Technik langsamer aufeinander zu, wenn es zum Anschlag kommt. Das bei Prothesenfüßen entstehende Geräusch wird durch die erfindungsgemäße Ausgestaltung verhindert oder zumindest so weit abgemildert, dass es nicht mehr störend oder vorzugsweise gar nicht mehr wahrgenommen wird. Die Erfindung betrifft also insbesondere eine Verwendung eines Reibelementes in einem Prothesenfuß der oben beschriebenen Art, um ein Reibmoment aufzubringen, das einem Verschwenken des Fußteiles relativ zu dem Unterschenkelteil entgegenwirkt, wenn das Fußteil relativ zu dem Unterschenkelteil arretiert ist. Die Erfindung ist folglich auch dann von Vorteil, wenn die Verbindung zwischen dem Fußteil und dem Unterschenkelteil zusätzlich mit einem weiteren Spiel, beispielsweise entlang der Schwenkachse, behaftet ist. Dieses zusätzliche Spiel kann, muss aber nicht, durch das Reibelement beeinflusst oder gar ausgeglichen werden.

Vorzugsweise ist das Reibmoment unabhängig von der Position, in der das Fußteil und das Unterschenkelteil zueinander arretiert sind und/oder unabhängig von der Schwenkrichtung, in der die durch das Spiel ermöglichte Bewegung erfolgt, konstant. Bei der vorliegenden Erfindung geht es insbesondere nicht darum, einen Schrittzyklus natürlicher wirken zu lassen, indem eine Dämpfung in die eine oder die andere Schwenkrichtung aufgebracht wird. Das aufgebrachte Reibmoment ist lediglich dazu vorgesehen, die durch das Spiel mögliche und daher eigentlich unerwünschte Bewegung nicht vollständig zu unterdrücken oder unmöglich zu machen, sondern dazu, diese eigentlich unerwünschte Bewegung zu verlangsamen, damit unerwünschte Geräusche nicht auftreten oder zumindest in ihrer Lautstärke abgeschwächt werden.

Die durch das Spiel ermöglichte Verschwenkung des Fußteils relativ zu dem Unterschenkelteil ist vorzugsweise kleiner als 1°, bevorzugt kleiner al 0,5°, besonders bevorzugt kleiner als 0,25°.

Das Reibmoment wird durch das wenigstens eine Reibelement aufgebracht, das zwischen dem Fußteil und dem Unterschenkelteil angeordnet ist. Dabei bedeutet die Anordnung "zwischen" den beiden Bauteilen insbesondere, dass eine Bewegung des einen der beiden Bauteile relativ zu anderen der beiden Bauteile das Reibmoment hervorruft. Es ist nicht notwendig, allerdings von Vorteil, dass sich das wenigstens eine Reibelement geometrisch zwischen dem Fußteil und dem Unterschenkelteil befindet. Dabei ist es vorzugsweise ausreichend, wenn sich ein Teil des wenigstens einen Reibelementes, besonders bevorzugt der Teil, der das Reibelement hervorruft, geometrisch zwischen dem Fußteil und dem Unterschenkelteil angeordnet ist. Vorzugsweise befindet sich das Fußteil vollständig auf einer Seite des wenigstens einen Reibelementes und das Unterschenkelteil vollständig auf der gegenüberliegenden Seite des wenigstens einen Reibelementes. Es ist jedoch ausreichend, wenn sich ein Teil des Fußteiles auf einer Seite des wenigstens einen Reibelementes befindet und ein Teil des Unterschenkelteiles sich auf der gegenüberliegenden Seite befindet.

Durch die erfindungsgemäße Ausgestaltung wird es zudem ermöglicht, die Arretierung funktional und/oder gegenständlich von dem Reibelement zu trennen. Das Reibelement ist vorzugsweise unabhängig von der Art der Arretierung, also insbesondere ein Element oder Bauteil, das für die Arretierung keine Rolle spielt. Um das Fußteil relativ zu dem Unterschenkelteil zu verstellen, also in einem anderen Schwenkwinkel zu arretieren, muss vorzugsweise das wenigstens eine Reibelement nicht gehandhabt, insbesondere nicht gelöst oder entfernt werden. Bevorzugt bringt das wenigstens eine Reibelement das Reibmoment auch dann auf, wenn das Fußteil relativ zu dem Unterschenkelteil bewegt, insbesondere verschwenkt wird, wenn das Fußteil nicht relativ zu dem Unterschenkelteil arretiert ist. In diesem Zustand ist die Bewegungsfreiheit der beiden Bauteile nicht durch das Spiel begrenzt.

Vorzugsweise ist das Reibelement eingerichtet, das Reibmoment unabhängig davon zu erzeugen, ob das Fußteil relativ zu dem Unterschenkelteil arretiert ist, so dass das Unterteil auch in diesem Zustand gegen das Reibmoment relativ zu dem Fußteil verschwenkt werden muss. Das Reibmoment ist vorzugsweise klein, beispielsweise kleiner als 5 Nm, bevorzugt kleiner als 2,5 Nm, besonders bevorzugt kleiner als 2,0 Nm, und größer als 0,5 Nm, bevorzugt größer als 1,0 Nm und besonders bevorzugt größer als 1,5 Nm. Ein solches Reibmoment ist auch bei der Verstellung des Schwenkwinkels, bei dem das Fußteil relativ zu dem Unterschenkelteil arretiert wird, durch den Träger des Prothesenfußes leicht zu überwinden.

Vorzugsweise ist das Unterschenkelteil durch eine formschlüssige Verbindung relativ zu dem Fußteil arretierbar. Dabei weisen bevorzugt das Unterschenkelteil und das Fußteil zueinander korrespondierend ausgebildete Formschlusselemente auf. Besonders bevorzugt ist an wenigstens einem dieser Formschlusselemente wenigstens ein Reibelement angeordnet. Dabei ist das wenigstens eine Reibelement bevorzugt derart an dem Formschlusselement angeordnet, dass es im arretierten Zustand der beiden Bauteile mit dem jeweils anderen Formschlusselement und/oder dem jeweils anderen Bauteil in Kontakt kommt und durch mechanische Reibung das Reibmoment hervorruft.

Alternativ oder zusätzlich dazu weisen das Unterschenkelteil und das Fußteil Formschlusselemente auf, die zu einem separaten Formschlussbauteil korrespondierend ausgebildet sind, wobei wenigstens ein Reibelement an dem Formschlussbauteil und/oder an wenigstens einem der Formschlusselemente angeordnet ist. Besonders bevorzugt ist das wenigstens eine Reibelement an allen Formschlusselementen angeordnet. Dafür ist es von Vorteil, wenn das wenigstens eine Reibelement mehrteilig ausgebildet ist und/oder der Prothesenfuß mehrere Reibelemente aufweist, von denen bevorzugt jeweils wenigstens eines an jedem der Formschlusselemente angeordnet ist. Die Formschlusselemente sind beispielsweise Bohrungen, die in dem Fußteil und dem Unterschenkelteil angeordnet sind. Die Bohrungen können als Durchgangsbohrung oder als Sackbohrung ausgebildet sein. Die Bohrungen werden bei den Schwenkwinkeln, bei denen das Fußteil an dem Unterschenkelteil arretiert werden soll, in Überdeckung gebracht. Dann wird das Formschlussbauteil, beispielsweise in Form eines Pins, eines Bolzens, eines Stiftes, einer Schraube oder einer Stange in die in Überdeckung befindlichen Bohrungen eingeführt und gegebenenfalls mit einem Sicherungselement, beispielsweise einer Schraube, gesichert.

Besonders bevorzugt ist das Formschlusselement derart ausgebildet, dass es manuell oder mechatronisch ausgerückt werden kann. In der ausgerückten Position kann die Absatzhöhe verstellt werden. Die Rückstellung in die Verriegelungsposition kann entweder manuell, mechanisch - beispielsweise über ein Federelement - oder mechatronisch erfolgen. Das Formschlusselement wird beim Ausrücken entlang einer Verschieberichtung verschoben und so mit dem korrespondierenden Formschlusselement außer Eingriff gebracht. Beim Einrücken oder auch Rückstellen wird das Formschlusselement in die entgegengesetzte Richtung verschoben und so mit dem korrespondierenden Formschlusselement wieder in Eingriff gebracht. Das zu verschiebende Formschlusselement ist bevorzugt als Zahnrad ausgebildet und die Verschieberichtung verläuft entlang der Rotationsachse des Zahnrades.

Das Formschlussbauteil ist bevorzugt an dem Unterschenkelteil oder dem Fußteil montierbar. Das Formschlussbauteil, das beispielsweise in die beschriebenen Bohrungen eingeführt ist, wird in diesem Fall vorzugsweise mit dem Fußteil oder dem Unterschenkelteil drehfest verbunden, so dass es mit dem jeweiligen Teil, an dem es montiert ist, relativ zu dem jeweils anderen Teil verschwenkbar ist. Insbesondere in diesem Fall ist es von Vorteil, wenn das wenigstens eine Reibelement an dem Formschlussbauteil angeordnet ist.

Erfindungsgemäß ist das wenigstens eine Reibelement an der Schwenkachse angeordnet. Erfindungsgemäß ist es um die Schwenkachse herum angeordnet. Dadurch werden konstruktiv besonders einfache Ausgestaltungen möglich. Das wenigstens eine Reibelement ist beispielsweise ringförmig ausgebildet, wobei die Schwenkachse, die in diesem Fall als Welle ausgebildet ist, durch die vorzugsweise mittige Öffnung des ringförmigen Reibelementes geführt ist. Aufgrund der Rotationssymmetrie um die Schwenkachse kann ein gleichförmiges und konstantes Reibmoment erreicht werden unabhängig von der Position und Orientierung und insbesondere unabhängig vom Schwenkwinkel des Fußteils relativ zu dem Unterschenkelteil. Da das Fußteil und das Unterschenkelteil bevorzugt nicht im Bereich der Schwenkachse relativ zueinander arretiert werden, stört ein in diesem Bereich angeordnetes Reibelement nicht, wenn die Arretierung gelöst und bei einem anderen Schwenkwinkel wieder erstellt wird.

In einer bekannten nicht beanspruchten Ausgestaltung eines Prothesenfußes ist die Schwenkachse eine virtuelle Schwenkachse. Das bedeutet insbesondere, dass es kein Bauteil des Prothesenfußes gibt, das als Welle wirkt, um die das Fußteil relativ zu dem Unterschenkelteil verschwenkbar ist. Dadurch kann die Schwenkachse weit in den Bereich des Prothesenfußes hineinverlegt werden, wodurch Bewegungsmuster des Fußes verändert und oftmals verbessert werden können. Um dies zu erreichen weisen das Fußteil und das Unterschenkelteil beispielsweise zueinander korrespondierend ausgebildete Formschlusselemente auf. Dies kann eine Nut und eine dazu korrespondierend ausgebildete Feder sein, die beide kreisbogenförmig geschwungen sind. Da die Nut und die Feder dabei den gleichen Krümmungsradius aufweisen, kann das Fußteil relativ zu dem Unterschenkelteil verschwenkt werden, indem die Feder in der Nut verschoben wird. Die Schwenkachse, die in diesem Fall eine virtuelle Schwenkachse ist, verläuft durch den Mittelpunkt des Kreisbogens.

Vorzugsweise weist das wenigstens eine Reibelement eine Beschichtung einer Lagerfläche des Fußteiles und/oder einer Lagerfläche des Unterschenkelteils und/oder einer Lagerfläche eines dritten Bauteiles, beispielsweise einer Welle oder dem Formschlussbauteil, auf. In einer nicht beanspruchten Ausgestaltung weist das wenigstens eine Reibelement zusätzlich wenigstens ein Dämpfungselement, insbesondere ein hydraulisches Dämpfungselement, auf. In diesem Fall verfügt der Prothesenfuß beispielsweise ein mit einem Fluid, insbesondere einem Hydrauliköl gefülltes Volumen auf. Dieses Volumen ist vorzugsweise an dem Fußteil oder dem Unterschenkelteil angeordnet und bevorzugt vollständig mit dem Fluid gefüllt. An dem jeweils anderen Bauteil, also dem Unterschenkelteil oder dem Fußteil ist ein Schieber drehfest angeordnet, der in das fluidgefüllte Volumen hineinragt. Wird nun das Fußteil relativ zu dem Unterschenkelteil verschwenkt, wird der Schieber in dem Volumen bewegt. Das Fluid stellt dieser Bewegung einen Strömungswiderstand entgegen, der von der Viskosität und den geometrischen Gegebenheiten abhängt. Dies betrifft etwa das Verhältnis der Querschnittsfläche des Volumens relativ zu der Querschnittsfläche des Schiebers in einer Richtung senkrecht zur Schwenkrichtung. Je größer die Viskosität des Fluids und je ähnlicher die Querschnittsfläche des Schiebers der des Volumens ist, desto größer ist der Widerstand und damit auch das Reibmoment.

Auch wenn ein solcher hydraulischer Dämpfer keine mechanische Reibung im Sinne zweier aneinander reibender Festkörper erzeugt, werden bevorzugt auch diese hydraulischen Momente und Kräfte als Reibmoment im Sinne der vorliegenden Erfindung verstanden.

In einer nicht beanspruchten Ausgestaltung hat das Reibelement auf ein in axialer Richtung vorhandenes Spiel zwischen dem Unterschenkelteil und dem Fußteil keinen Einfluss.

Mithilfe der beiliegenden Zeichnungen werden nachfolgend einige Ausführungsbeispiele der vorliegenden Erfindung näher erläutert. Es zeigen
- Figuren 1 bis 4 -: schematische Schnittdarstellungen durch Teile von Prothesenfüßen gemäß einigen Ausführungsbeispielen der vorliegenden Erfindung,
- Figuren 5 und 6 -: schematische Darstellungen des Knöchelmomentes im Verlauf eines Schrittzyklus,
- Figuren 7 und 8 -: schematische Schnittdarstellungen durch Teile von Prothesenfüßen gemäß weitere Ausführungsbeispielen der vorliegenden Erfindung
- Figur 9 -: die schematische Darstellung eines Prothesenfußes gemäß einem Ausführungsbeispiel der vorliegenden Erfindung,
- Figur 10 -: die schematische Darstellung eines weiteren Prothesenfußes,
- Figur 11 -: die schematische Darstellung zweier Bauteile eines Prothesenfußes und
- Figuren 12 und 13: schematische Darstellungen eines Teiles eine Prothesenfußes.

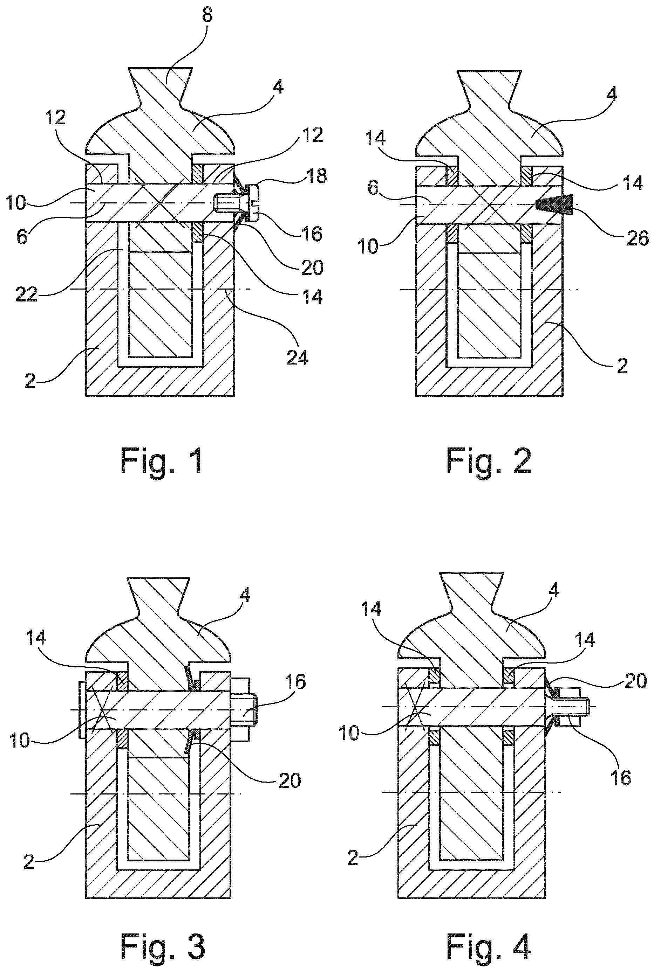

Figur 1 zeigt eine Schnittdarstellung durch einen Teil eines Prothesenfußes einen Fußteil 2, von dem nur ein Befestigungselement dargestellt ist, ist mit einem Unterschenkelteil 4 um eine Schwenkachse 6 schwenkbar verbunden. Vom Unterschenkelteil 4 ist nur ein Teil mit einem Adapter 8 gezeigt, an dem ein weiteres Prothesenteil befestigbar ist. In Figur 1 ist das Unterschenkelteil 4 mit einer Welle 10 drehfest verbunden, innerhalb derer die Schwenkachse 6 verläuft und die in 2 dafür vorgesehenen Aufnahmen 12 im Fußteil 2 drehbar gelagert ist. Zwischen dem Unterschenkelteil 4 und der rechten Seite des Fußteils 2 befindet sich in Figur 1 ein Reibelement 14, das in der gezeigten Ausführungsform um die Welle 10 herum angeordnet ist. In der Welle 10 ist in einer dafür vorgesehenen Gewindebohrung eine Schraube 16 angeordnet, die einen Schraubenkopf 18 aufweist. Zwischen dem Schraubenkopf 18 und der Außenseite des Fußteils 2 befindet sich eine Tellerfeder 20, die komprimiert und damit energetisch aufgeladen werden kann, indem die Schraube 16 in die Welle 6 eingeschraubt werden kann. Die Tellerfeder 20 stützt sich an der Außenseite des Fußteils 2 ab, sodass das auf der Welle 10 befestigte Unterschenkelteil 4 durch die Schraube 16 und die Tellerfeder 20 in Richtung auf den rechten Schenkel des dargestellten Elementes des Fußteils 2 gezogen wird und so das Reibelement 14 komprimiert wird. Das vom Reibelement 14 aufgebracht Reibmoment ist auf diese Weise einstellbar.

Das Reibmoment, das vom Reibelement 14 aufgebracht wird, wirkt dabei einer Verschwenkung des Fußteils 2 relativ zum Unterschenkelteil 4 entgegen. Ein in axialer Richtung bezüglich der Schwenkachse 6 vorhandenes Spiel zwischen dem Fußteil 2 und dem Unterschenkelteil 4, das durch den Spalt 22 dargestellt wird, wird durch das Reibelement 14 im gezeigten Ausführungsbeispiel nicht beeinträchtigt. Entlang der eingezeichneten Linie 24 kann ein Arretierelement das Fußteil 2 und das Unterschenkelteil 4 relativ zueinander arretieren, wobei jedoch ein Spiel in Schwenkrichtung um die Schwenkachse 6 herum bleibt.

Figur 2 zeigt eine ähnliche Ausgestaltung, bei der das Unterschenkelteil 4 drehfest mit der Welle 10, in der die Achse 6 verläuft, verbunden ist. Zwischen den beiden Schenkeln des Fußteils 2 sind in Figur 2 im Unterschied zur in Figur 1 gezeigten Ausgestaltung zwei Reibelemente 14 angeordnet. Anstelle der Schraube 16 mit der unter dem Schraubenkopf 18 angeordneten Tellerfeder 20 ist auf der rechten Seite in die Welle 10 ein Keil 26 eingebracht, der die Welle 10 an dieser Stelle aufweitet und so für die nötige Kraftübertragung sorgt.

Figur 3 zeigt ein Fußteil 2, das drehfest mit der Welle 10 verbunden ist. Auf dieser Welle 10 befindet sich drehbar gelagert das Unterschenkelteil 4. Zwischen dem linken Schenkel des Fußteils 2 und dem Unterschenkelteil 4 befindet sich ein Reibelement 14, während sich auf der gegenüberliegenden Seite eine Tellerfeder 20 befindet. Diese wird durch die durch die Welle 10 hindurchführende Schraube 16 gespannt und komprimiert und sorgt so für die nötige Kraft.

Figur 4 zeigt eine zu Figur 3 ähnliche Ausgestaltung, bei der das Fußteil 2 drehfest mit der Welle 10 verbunden ist, auf der das Unterschenkelteil 4 drehbar gelagert ist. Auf beiden Seiten des Unterschenkelteils 4 ist jeweils ein Reibelement 14 angeordnet, die durch die von der Schraube 16 und der Tellerfeder 20 aufgebrachten Kraft komprimiert werden.

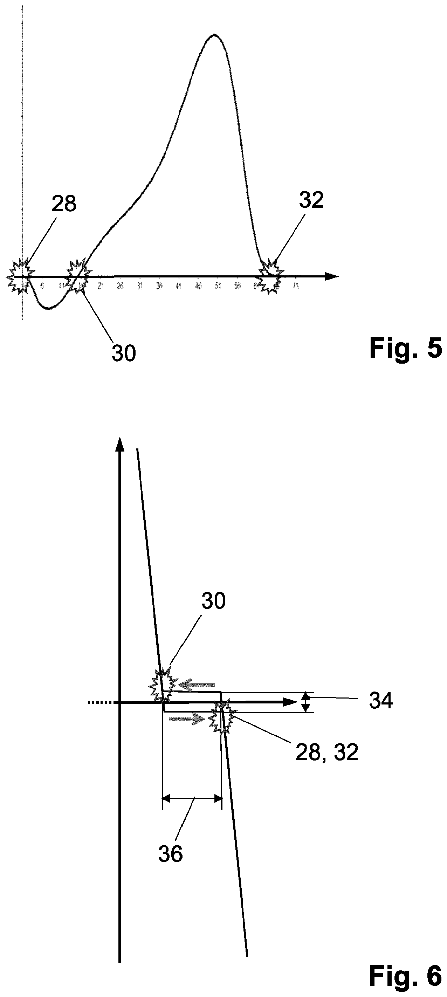

Figur 5 zeigt ein schematisches Diagramm, das den Verlauf des Knöchelmomentes (y-Achse) über den Schrittzyklus (x-Achse) zeigt. Der Zyklus beginnt mit dem Fersenauftritt, ab dem ein negatives Moment anliegt, das auf den Knöchel in Plantarflexionsrichtung wirkt. Dabei schlagen aufgrund des vorhandenen Spiels das Fußteil und das Unterschenkelteil aneinander an, was durch die Wolke 28 gekennzeichnet ist. Im Laufe des Schrittes nimmt das Moment zunächst an Stärke zu, was in Figur 5 dazu führt, dass es stärker negativ wird. Ab dem Moment, in dem der Fuß vollständig auf dem Boden aufsetzt, wirkt ein positives Moment, das auf den Knöchel in Dorsalflexionsrichtung wirkt. Beim Übergang wechselt das Moment das Vorzeichen und es kommt zum Nulldurchgang, der durch die Wolke 30 gekennzeichnet ist. Da die Richtung des anliegenden Momentes wechselt, kommt es erneut zu einem Anschlag zwischen dem Fußteil und dem Unterschenkelteil, der bei Prothesenfüßen gemäß dem Stand der Technik mit einem Anschlagsgeräusch verbunden ist. Dieses Geräusch wird bei der vorliegenden Erfindung durch das Reibmoment verringert oder sogar ganz verhindert.

Erst wenn der Fuß den Boden wieder verlässt (toe-off) ist das Moment wieder Null und es kommt zum dritten Anschlag des Fußteils und des Unterschenkelteils, der durch die Wolke 32 gekennzeichnet ist.

Figur 6 zeigt eine schematische Vergrößerung bei den jeweiligen Anschlägen. Bei den durch die Wolken 28, 32 gekennzeichneten Anschlägen verläuft die Kurve des Momentes von links nach rechts, also von positivem Moment zu negativem Moment. Dabei muss das wirkende Moment leicht negativ werden, bevor es zu der durch das Spiel ermöglichten Bewegung zwischen dem Fußteil und dem Unterschenkelteil kommt. Der Abstand 34 entspricht dabei dem doppelten Reibmoment. Kommt es zu der Bewegung, rutscht der Winkel zwischen dem Fußteil und dem Unterschenkelteil um einen Betrag nach rechts, was durch den Abstand 36 dargestellt ist. Dieser Abstand entspricht dem Spiel. Bei dem durch die Wolke 30 gekennzeichneten Anschlag verläuft das Moment umgekehrt.

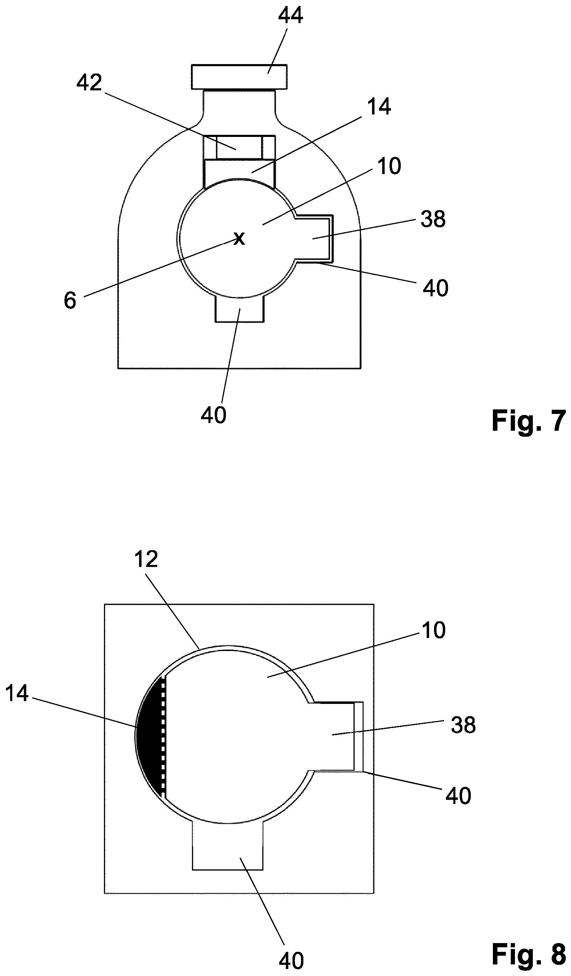

Figur 7 zeigt eine Schnittdarstellung durch einen Teil eines Prothesenfußes senkrecht zur Schwenkachse 6. Diese verfügt über einen Vorsprung 38, der in 2 dafür vorgesehene Ausnehmungen 40 einbringbar ist. Ein Reibelement 14 ist so angeordnet, dass es mit der Welle 10 in Kontakt kommt und so ein Reibmoment aufbringt. Über eine Schraube 42, die mit einem Betätigungselement 44 verbunden ist, kann ein Druck eingestellt werden, den das Reibelement 14 auf die Welle 10 ausübt. Damit ist auch das Reibmoment einstellbar.

Figur 8 zeigt eine ähnliche Darstellung. Die Welle 10 verfügt über den Vorsprung 38, der in die Ausnehmungen 40 einbringbar ist. Auf der dem Vorsprung 38 gegenüberliegenden Seite der Welle 10 ist ein Reibelement 14 dargestellt, dass an der Innenseite der Aufnahme 12, in der die Welle 10 gelagert ist, ein Reibmoment aufbringt.

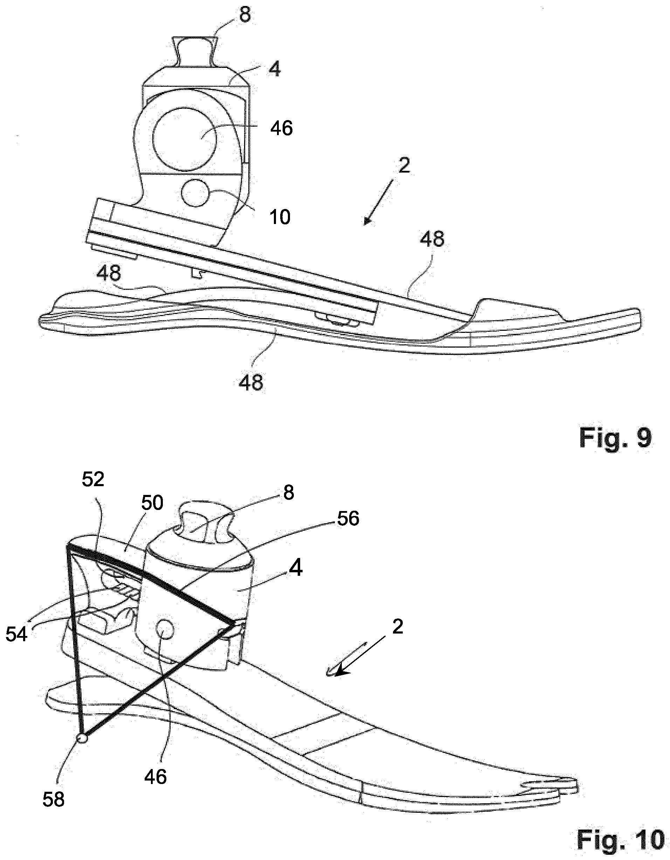

Figur 9 zeigt einen Prothesenfuß mit dem Fußteil 2 und dem Unterschenkelteil 4, an dem sich ein Adapter 8 befindet. Über die Welle 10 kann das Fußteil 2 relativ zum Unterschenkelteil 4 verschenkt werden. Ein Formschlusselement 46, das in Figur 9 nicht dargestellt ist und das beispielsweise drehfest mit dem Unterschenkelteil 4 verbunden ist, kann beispielsweise durch Betätigen eines Betätigungselementes, beispielsweise durch Drücken eines Knopfes, entlang einer Verschieberichtung verschoben werden, die in der in Figur 9 gezeigten Ausführungsform senkrecht auf der Zeichenebene steht. Im eingerückten Zustand greift das Formschlusselement in ein korrespondierend ausgebildetes zweites Formschlusselement ein, das drehfest am Fußteil befestigt ist. Aufgrund des Spiels zwischen dem Unterschenkelteil 4 und dem Fußteil 2 und den jeweiligen Formschlusselementen ist dennoch eine kleine Bewegung möglich. Das Fußteil 2 verfügt zudem über verschiedene Federn 48, die für die nötige Elastizität sorgen. Das Reibelement 14 ist in der gezeigten Darstellung nicht erkennbar. Vorzugsweise ist das auszurückende Formschlusselement in Richtung der eingerückten Position federbelastet.

Figur 10 zeigt eine weitere Ausführungsform eines Prothesenfußes mit dem Fußteil 2 und dem Unterschenkelteil 4, an dem sich der Adapter 8 befindet. Am Fußteil 2 befindet sich eine Schiene 50, an der das Unterschenkelteil 4 entlanggleiten kann. Sie verfügt über ein Langloch 52, das über mehrere Rillen 54 verfügt, die als unterschiedliche Rastpositionen für das Unterschenkelteil 4 an der Schiene 50 dienen. In jeder dieser Rillen 54 kann das Formhsclusselement46, das in diesem Fall beispielsweise als Pin ausgebildet ist, befestigt werden, wodurch das Unterschenkelteil 4 in der jeweiligen Position arretiert wird. Dazu kann der Pin 46 beispielsweise manuell oder mechatronisch ausgerückt und so mit den Rillen 54 außen Eingriff gebracht werden. Danach kann die Position des Unterschenkelteiles 4 relativ zu dem Fußteil 2 verschoben und angepasst werden. Danach wird der Pin 46 wieder eingerückt und so wieder mit einer der Rillen 54 in Eingriff gebracht. Im gezeigten Ausführungsbeispiel ist das Unterschenkelteil 4 folglich entlang der Linie 56 verschiebbar, die kreisbogenförmig ausgebildet ist und einen virtuellen Drehpunkt 58 aufweist.

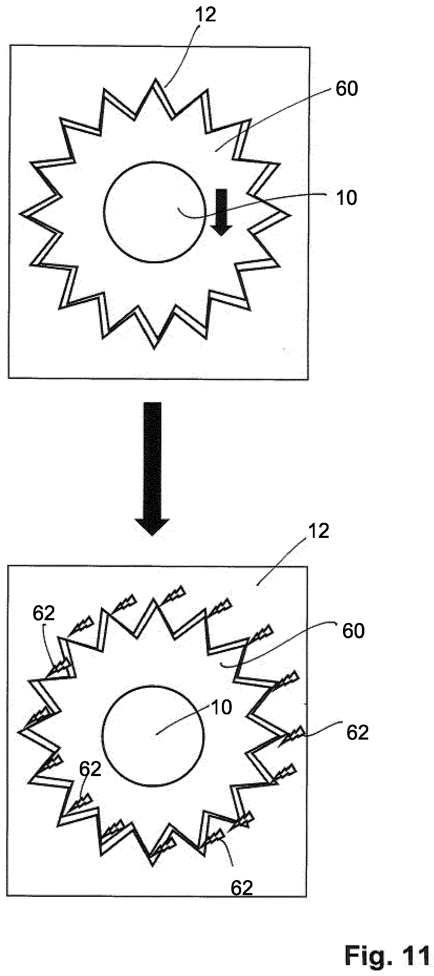

Figur 11 zeigt schematisch, wodurch dass durch die vorliegende Erfindung zu lösende Problem entsteht. Man erkennt schematisch ein Zahnrad 60, das im gezeigten Ausführungsbeispiel drehfest mit der Welle 10 verbunden ist. Die Kombination aus Zahnrad 60 und Welle 10 ist in einer Aufnahme 12 angeordnet, die als Hohl-Zahnrad ausgebildet ist. Die Aufnahme ist mit dem Unterschenkelteil 4 oder dem Fußteil 2 und das Zahnrad 60 mit dem jeweils anderen Teil verbunden.

Im oberen Teil der Figur 11 ist das Zahnrad 60 mit der Welle 10 gegen den Uhrzeigersinn mit einem Drehmoment belastet, sodass die jeweils gegen den Uhrzeigersinn weisenden Flanken der einzelnen Zähne des Zahnrades 60 an den jeweiligen Flanken der nach innen ragenden Zähne der Aufnahme 12 anliegen. Beim Übergang zum unteren Teil der Figur 11 ändert sich die Belastungsrichtung und das Drehmoment, das auf die Welle 10 wirkt, wechselt das Vorzeichen. Unter diesem Drehmoment wird das Zahnrad 60 mit der Welle 10 im Uhrzeigersinn belastet. Aufgrund des Spiels zwischen dem Zahnrad 60 und der Aufnahme 12 ist eine minimale Bewegung der beiden Bauteile relativ zueinander möglich, bis die im unteren Teil der Figur 11 gezeigte Situation erreicht ist. In dieser liegen die in Richtung des Uhrzeigersinns weisenden Flanken der Zähne des Zahnrades 60 an den nach innen ragenden Zähnen der Aufnahme 12 an. Da zwischen der oberen Situation aus Figur 11 und der unteren Situation eine minimale Bewegung stattgefunden hat, entsteht an jeder Stelle, wo ein Zahn des Zahnrades 60 an die Aufnahme 12 anschlägt, ein Geräusch, das durch die Pfeile 62 angedeutet ist.

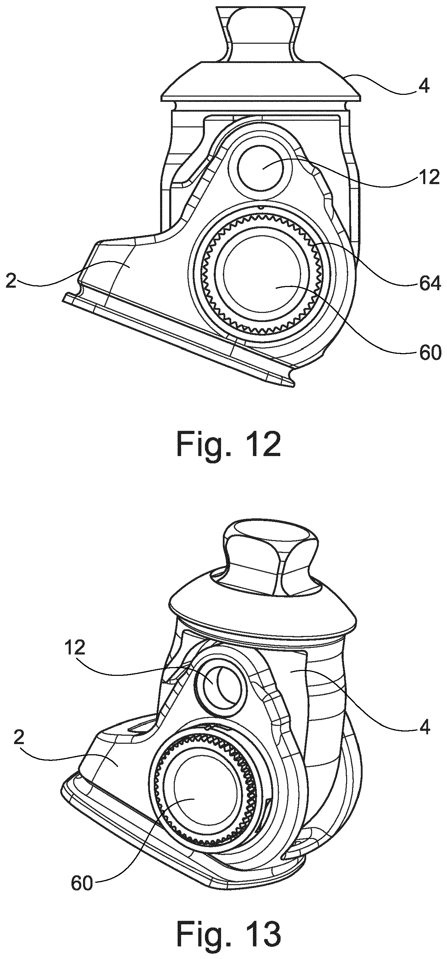

Figur 12 und 13 zeigen zwei Darstellungen eines Gelenkes eines Prothesenfußes gemäß eines Ausführungsbeispiels der vorliegenden Erfindung. Man erkennt einen Teil des Unterschenkelelementes 4 und des Fußteiles 2, die um eine Schwenkachse schwenkbar aneinander angeordnet sind. Sie sind auf einer nicht dargestellten Welle 10 angeordnet, die in die in Figur 12 gezeigte Aufnahme 12 eingesteckt wird. In Figur 12 ist das Zahnrad 60 im eingerückten Zustand gezeigt. Es greift in ein korrespondierend ausgebildetes Formschlusselement 64 ein, das wie in Figur 11 als Aufnahme ausgebildet ist. Es entsteht die in Figur 11 gezeigte Situation.

Figur 13 hingegen zeigt das Zahnrad 60 in ausgerückter Position. Es ist entlang eines Verschieberichtung, die in Figur 13 parallel zu der Aufnahme 12 verläuft, durch dir die Welle geschoben werden kann, verschoben und so mit dem korrespondierend ausgebildeten Formschlusselement 64 außer Eingriff gebracht worden.

### Bezugszeichenliste

- 2: Fußteil
- 4: Unterschenkelteil
- 6: Schwenkachse
- 8: Adapter
- 10: Welle
- 12: Aufnahme
- 14: Reibelement
- 16: Schraube
- 18: Schraubenkopf
- 20: Tellerfeder
- 22: Spalt
- 24: Linie
- 26: Keil
- 28: Wolke
- 30: Wolke
- 32: Wolke
- 34: Abstand
- 36: Abstand
- 38: Vorsprung
- 40: Ausnehmung
- 42: Schraube
- 44: Betätigungselement
- 46: Formschlusselement
- 48: Feder
- 50: Schiene
- 52: Langloch
- 54: Rille
- 56: Linie
- 58: virtueller Drehpunkt
- 60: Zahnrad
- 62: Pfeil
- 64: korrespondierendes Formschlusselement

## Patentansprüche

1. Prothesenfuß mit
- einem Fußteil (2) und
- einem Unterschenkelteil (4), das um eine Schwenkachse (6) schwenkbar mit dem Fußteil (2) verbunden und in verschiedenen Schwenkwinkeln relativ zu dem Fußteil (2) arretierbar ist,
wobei die Verbindung zwischen dem Unterschenkelteil (4) und dem Fußteil (2) mit einem Spiel in Schwenkrichtung behaftet ist, wenn das Fußteil (2) an dem Unterschenkelteil (4) arretiert ist,
wobei der Prothesenfuß wenigstens ein Reibelement (14) aufweist, das zwischen dem Fußteil (2) und dem Unterschenkelteil (4) an der Schwenkachse (6) angeordnet ist und ein Reibmoment aufbringt, das einem Verschwenken des Fußteiles (2) relativ zu dem Unterschenkelteil (4) entgegenwirkt, wenn das Fußteil (2) relativ zu dem Unterschenkelteil (4) arretiert ist, **dadurch gekennzeichnet, dass** das wenigstens eine Reibelement (14) um die Schwenkachse (6) herum angeordnet ist.

2. Prothesenfuß nach Anspruch 1, **dadurch gekennzeichnet, dass** das Reibelement (14) eingerichtet ist, das Reibmoment unabhängig davon zu erzeugen, ob das Fußteil (2) relativ zu dem Unterschenkelteil (4) arretiert ist, so dass das Unterteil gegen das Reibmoment relativ zu dem Fußteil (2) verschwenkt werden muss.

3. Prothesenfuß nach Anspruch 1 oder 2, **dadurch gekennzeichnet, dass** das Unterschenkelteil (4) durch eine formschlüssige Verbindung relativ zu dem Fußteil (2) arretierbar ist.

4. Prothesenfuß nach Anspruch 3, **dadurch gekennzeichnet, dass** das Unterschenkelteil (4) und das Fußteil (2) zueinander korrespondierend ausgebildete Formschlusselement aufweist, wobei an wenigstens einem dieser Formschlusselemente wenigstens ein weiteres Reibelement (14) angeordnet ist

5. Prothesenfuß nach Anspruch 3, **dadurch gekennzeichnet, dass** das Unterschenkelteil (4) und das Fußteil (2) Formschlusselemente aufweisen, die zu einem separaten Formschlussbauteil korrespondieren ausgebildet sind, wobei wenigstens ein weiteres Reibelement (14) an dem Formschlussbauteil und/oder an wenigstens einem der Formschlusselemente angeordnet ist.

6. Prothesenfuß nach Anspruch 5, **dadurch gekennzeichnet, dass** das Formschlussbauteil an dem Unterschenkelteil (4) oder dem Fußteil (2) montierbar ist.

7. Prothesenfuß nach einem der vorstehenden Ansprüche, **dadurch gekennzeichnet, dass** das wenigstens eine Reibelement (14) eine Beschichtung einer Lagerfläche des Fußteiles (2) und/oder einer Lagerfläche des Unterschenkelteils (4) und/oder einer Lagerfläche eines dritten Bauteiles, beispielsweise einer Welle (10) oder dem Formschlussbauteil, aufweist.

## Claims

1. A prosthetic foot with
- a foot part (2) and
- a lower leg part (4) that is connected to the foot part (2) such that it can be pivoted about a pivot axis (6) and that can be locked in various pivot angles relative to the foot part (2),
the connection between the lower leg part (4) and the foot part (2) having a clearance in the pivot direction when the foot part (2) is locked on the lower leg part (4),
wherein the prosthetic foot has at least one friction element (14) arranged between the foot part (2) and the lower leg part (4) on the pivot axis (6) and applies a frictional torque that counteracts a pivoting of the foot part (2) relative to the lower leg part (4) when the foot part (2) is locked relative to the lower leg part (4)
**characterized in that** the at least one friction element (14) is arranged about the pivot axis (6).

2. The prosthetic foot according to claim 1, **characterized in that** the friction element (14) is configured to generate the frictional torque independently of whether the foot part (2) is locked relative to the lower leg part (4), so that the lower part also has to be pivoted relative to the foot part (2) against the frictional torque.

3. The prosthetic foot according to claim 1 or 2, **characterized in that** the lower leg part (4) can be locked relative to the foot part (2) by way of a form-fitting connection.

4. The prosthetic foot according to claim 3, **characterized in that** the lower leg part (4) and the foot part (2) comprise form-fitting elements designed to correspond to each other, at least one additional friction element (14) being arranged on at least one of these form-fitting elements.

5. The prosthetic foot according to claim 3, **characterized in that** the lower leg part (4) and the foot part (2) feature form-fitting elements that are designed to correspond to a separate form-fitting component, wherein at least one additional friction element (14) is arranged on the form-fitting component and/or on at least one of the form-fitting elements.

6. The prosthetic foot according to claim 5, **characterized in that** the form-fitting component can be mounted on the lower leg part (4) or the foot part (2).

7. The prosthetic foot according to one of the preceding claims, **characterized in that** the at least one friction element (14) features a coating of a bearing surface of the foot part (2) and/or a bearing surface of the lower leg part (4) and/or a bearing surface of a third component, such as a shaft (10) or the form-fitting component.

## Revendications

1. Prothèse de pied comprenant
- une partie pied (2) et
- une partie jambe (4) reliée à la partie pied (2) de manière à pouvoir pivoter autour d'un axe de pivotement (6) et apte à être verrouillée par rapport à la partie pied (2) selon différents angles de pivotement,
la liaison entre la partie jambe (4) et la partie pied (2) présentant un jeu dans la direction de pivotement lorsque la partie pied (2) est verrouillée sur la partie jambe (4),
la prothèse de pied comportant au moins un élément de frottement (14) qui est disposé entre la partie pied (2) et la partie jambe (4) au niveau de l'axe de pivotement (6) et qui exerce un couple de frottement qui s'oppose à un pivotement de la partie pied (2) par rapport à la partie jambe (4) lorsque la partie pied (2) est verrouillée par rapport à la partie jambe (4),
**caractérisée en ce que**
ledit au moins un élément de frottement (14) est disposé autour de l'axe de pivotement (6).

2. Prothèse de pied selon la revendication 1,
**caractérisée en ce que** l'élément de frottement (14) est agencé pour générer le couple de frottement indépendamment du fait que la partie pied (2) soit verrouillée ou non par rapport à la partie jambe (4), de sorte que c'est à l'encontre du couple de frottement que la partie jambe doive pivoter par rapport à la partie pied (2).

3. Prothèse de pied selon la revendication 1 ou 2,
**caractérisée en ce que** la partie jambe (4) peut être verrouillée par rapport à la partie pied (2) au moyen d'une liaison par complémentarité de forme.

4. Prothèse de pied selon la revendication 3,
**caractérisée en ce que** la partie jambe (4) et la partie pied (2) comportent des éléments de liaison par complémentarité de forme conçus pour correspondre l'un à l'autre, au moins un élément de frottement supplémentaire (14) étant disposé sur l'un au moins de ces éléments de liaison par complémentarité de forme.

5. Prothèse de pied selon la revendication 3,
**caractérisée en ce que** la partie jambe (4) et la partie pied (2) comportent des éléments de liaison par complémentarité de forme conçus pour correspondre à un composant de liaison par complémentarité de forme distinct, au moins un élément de frottement supplémentaire (14) étant disposé sur le composant de liaison par complémentarité de forme et/ou sur l'un au moins des éléments de liaison par complémentarité de forme.

6. Prothèse de pied selon la revendication 5,
**caractérisée en ce que** le composant de liaison par complémentarité de forme peut être monté sur la partie jambe (4) ou sur la partie pied (2).

7. Prothèse de pied selon l'une des revendications précédentes,
**caractérisée en ce que** ledit au moins un élément de frottement (14) comprend un revêtement d'une surface d'appui de la partie pied (2) et/ou d'une surface d'appui de la partie jambe (4) et/ou d'une surface d'appui d'un troisième composant, par exemple d'un arbre (10) ou du composant de liaison par complémentarité de forme.
